# EUROPEAN PATENT SPECIFICATION

(11) **EP 3 203 434 B1**
(45) Date of publication and mention of the grant of the patent: **08.04.2020**
(21) Application number: 15845814.1
(22) Date of filing: 30.07.2015
(51) Int. Cl.: G16H 10/40, C12M 1/36

(54) **DEVICE, METHOD AND PROGRAM FOR ACQUIRING INFORMATION ON CELLS**
VORRICHTUNG, VERFAHREN UND PROGRAMM ZUR ERFASSUNG VON INFORMATIONEN ÜBER ZELLEN
DISPOSITIF, PROCÉDÉ ET PROGRAMME D'ACQUISITION D'INFORMATIONS SUR DES CELLULES

(30) Priority: 29.09.2014 JP 2014198474
(43) Date of publication of application: 09.08.2017
(73) Proprietor: Fujifilm Corporation, Minato-ku Tokyo 106-8620 (JP)
(72) Inventor: MATSUMOTO, Tsuyoshi, Kanagawa 258-8538 (JP)
(74) Representative: Klunker IP Patentanwälte PartG mbB
(86) International application number: PCT/JP2015/071704
(87) International publication number: WO 2016/051946

(56) References cited:
- EP-A1- 2 202 291
- EP-A1- 2 213 722
- EP-A1- 2 453 005
- EP-A1- 2 584 029
- WO-A1-2013/047290
- WO-A2-2004/079328
- JP-A- 2007 068 486
- JP-A- 2008 293 151
- JP-A- 2012 147 693
- JP-A- 2012 170 358
- KR-A- 20130 077 957
- US-A1- 2009 299 763

## Description

### BACKGROUND OF THE INVENTION

### 1. Field of the Invention

The present invention relates to a management system including a cell culture apparatus and a cell information acquisition apparatus, a cell information acquisition method, and a cell information acquisition program that acquire information relating to a tissue formed of a plurality of cells which is cultured.

### 2. Description of the Related Art

In recent years, regenerative medicine for culturing cells such as skin, retina or myocardium in a sheet shape and transplanting the cultured cells to a patient to treat a disease has attracted attention. Further, a technique for culturing cells such as blood corpuscles or a nerve used for regenerative medicine has been developed.

When producing such a cell sheet such as skin, first, a small amount of cells are picked from a living body, the picked cells are induced to undifferentiated cells such as iPS cells, and then, the obtained undifferentiated cells are cultured. Further, the obtained cells are differentiation-induced to skin cells, and the differentiation-induced cells are disposed in a sheet shape for culture to produce the cell sheet.

EP 2 202 291 A1 discloses an incubation container that a user can easily identify based on image analysis data. EP 2 453 005 A1 teaches enhancing work efficiency in a cell selection and extraction method. EP 2 213 722 A1 teaches to search, based on a correspondence relationship determined from the position and the shape of cells, a plurality of cell analyzing tables at different observing times in the direction of a time axis for each of the cells having commonality. KR 2013 0077957 A discloses a cell culture observation apparatus enabling to easily acquire information of a cell culture condition by analyzing the area of cells in a culture container and to test a material which affects a medium change time, a culture finish time, a cell survival rate, and cell migration. EP 2 584 029 A1 discloses a system by which a dissociation process of cells constituting a sheet-shaped cell culture is carried out in a simple and assured way. US 2009/079328 A2 discloses conducting a stem cell technology based on induced pluripotent stem cells (iPSCs) and cells differentiated from iPSCs implemented by means of a database of iPSC-derived cells that is used for tracking customers and samples. WO 2004/079328 A2 addresses linking information from a donor with a biological sample and/or information related to that sample and providing valuable information relating to a biological sample to the donor of that sample.

### SUMMARY OF THE INVENTION

Here, when forming a cell sheet or the like by disposing a plurality of cells as described above, there is not only a case where cells cultured under the same culture environment or the same culture conditions are necessarily used, but also, a case where cells cultured under different culture lines or culture conditions are collected to form a cell sheet, for example.

In such a case, for example, in a case where there is a problem in a tissue such as a cell sheet transplanted to a predetermined patient, it is necessary to specify a culture line in which cells used to form the cell sheet are cultured, or specify a culture environment or culture conditions under which cells used to form the cell sheet are cultured, and it is necessary to find causes, to perform an improvement action, or to perform minimum recovery of cells, for example.

That is, it is necessary to secure traceability of cells used for formation of a tissue such as a cell sheet. As a method for managing cell culture information, for example, a technique in which a radio frequency identifier (RFID) chip in which identification information is stored is provided on a culture vessel, and the identification information read from the RFID chip and culture media exchange history information are stored in association for management is disclosed in JP2011-19452A. Further, JP2014-124107A discloses a technique in which an integrated circuit (IC) tag is provided on a culture vessel, identification information, culture setting information, and the like are stored in the IC tag, and the information is read for management.

That is, JP2011-19452A and JP2014-124107A disclose a technique for individually managing culture information about cells in a culture vessel unit, but when forming a tissue using the cells, loss of the managed information may easily occur. Accordingly, when forming a tissue, it is not possible to secure traceability.

In order to solve the above-mentioned problems, an object of the invention is to provide a management system including a cell culture apparatus and a cell information acquisition apparatus, a cell information acquisition method, and a cell information acquisition program capable of securing, when forming a tissue or the like using a plurality of cells as described above, traceability of culture information or the like of cells that form the tissue.

According to an aspect of the invention, there is provided a management system including a cell culture apparatus and a cell information acquisition apparatus according to claim 1.

In the cell information acquisition apparatus, the integrated information acquisition unit may acquire the integrated information by integrating the entirety of the additional information assigned to the respective cell culture units.

The integrated information acquisition unit may acquire the integrated information by classifying the additional information based on dates.

The partial information may include a culture condition of each cell culture unit.

The partial information may include a retrieval index used for retrieving the additional information of each cell culture unit.

The partial information may include information relating to a specific date that is set in advance.

The integrated information acquisition unit may acquire the integrated information by processing the additional information assigned to the respective cell culture units.

The integrated information acquisition unit may acquire the integrated information by compressing the additional information assigned to the respective cell culture units.

The integrated information acquisition unit may acquire the integrated information by converting the additional information assigned to the respective cell culture units into secondary information based on the additional information.

It is preferable that the secondary information is quality information about the integrative cell culture unit.

It is preferable that the secondary information is information capable of representing the additional information as a visual graph.

It is preferable that the secondary information is information relating to a temporal change.

The integrated information acquisition unit may cause information acquired with respect to the integrative cell culture unit to be included in the integrated information.

According to another aspect of the invention, there is provided a cell information acquisition method according to claim 14.

According to still another aspect of the invention, there is provided a cell information acquisition program according to claim 15.

According to the cell information acquisition apparatus, the cell information acquisition method and the cell information acquisition program of the invention, since when acquiring additional information assigned to a plurality of cell culture units and integrating cells which are respectively cultured in the plurality of cell culture units to form an integrative cell culture unit, the additional information of the respective cell culture units is integrated to acquire integrated information relating to the integrative cell culture unit, it is possible to secure traceability in each cell culture unit with reference to the integrated information.

### BRIEF DESCRIPTION OF THE DRAWINGS

Fig. 1 is a block diagram illustrating a schematic configuration of a cell information management system using a cell information acquisition apparatus according to an embodiment of the invention.
Fig. 2 is a diagram illustrating an example of a culture vessel.
Fig. 3 is a flowchart illustrating an operation of the cell information management system using the cell information acquisition apparatus according to the embodiment of the invention.
Fig. 4 is a schematic view illustrating a case where the entirety of additional information assigned to respective wells is integrated to acquire integrated information.
Fig. 5 is a schematic view illustrating a case where partial information among additional information assigned to respective wells is integrated to acquire integrated information.
Fig. 6 is a diagram illustrating an example of a table in which the type of a tissue and partial information to be integrated are associated with each other.
Fig. 7 is a schematic view illustrating a case where retrieval indexes used for retrieving additional information of respective wells are integrated to acquire integrated information.
Fig. 8 is a schematic view illustrating a case where additional information assigned to each well is compressed to acquire integrated information.
Fig. 9 is a schematic view illustrating a case where pieces of donor information which are repeated are connected and compressed.
Fig. 10 is a schematic view illustrating a case where a quality evaluation result assigned to each well is converted into a quality evaluation result having the highest frequency to acquire integrated information.
Fig. 11 is a schematic view illustrating a case where a quality evaluation result assigned to each well is converted into an average quality evaluation result to acquire integrated information.
Fig. 12 is a schematic view illustrating a case where additional information is converted into information capable of being displayed as a visual histogram to acquire integrated information.

### DESCRIPTION OF THE PREFERRED EMBODIMENTS

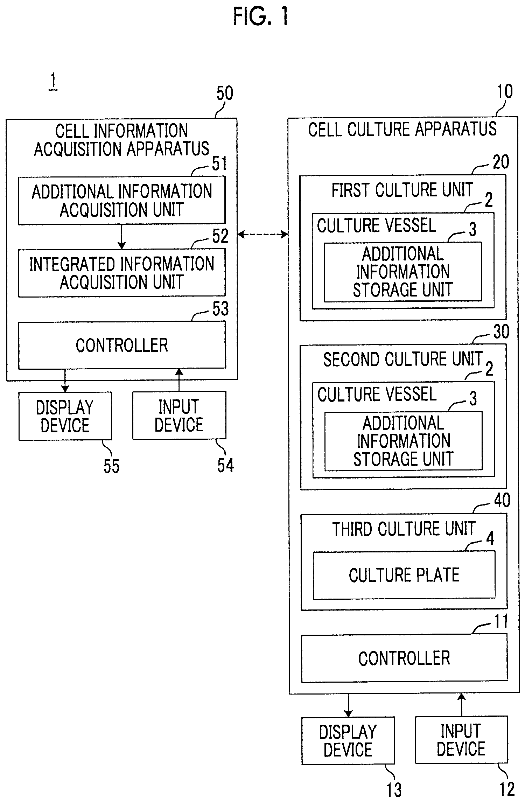

Hereinafter, a cell information management system using a cell information acquisition apparatus according to an embodiment of the invention will be described in detail with reference to the accompanying drawings. Fig. 1 is a block diagram illustrating a schematic configuration of a cell information management system 1 of this embodiment.

The cell information management system 1 of this embodiment includes a cell culture apparatus 10 and a cell information acquisition apparatus 50, as shown in Fig. 1.

The cell culture apparatus 10 includes first to third culture units 20, 30, and 40, and a controller 11. The first to third culture units 20, 30, and 40 perform culture of cells. As cells which are culture targets, for example, differentiation-induced cells that form a sheet-shaped tissue such as skin, retina or myocardium, differentiation-induced cells that form a tissue such as blood corpuscles, a nerve, or an organ, or the like, may be used. The cells which are the culture targets are not limited thereto, and any cells which are disposed in two dimensions to form a sheet-shaped tissue or which are disposed in three dimensions to form a predetermined tissue may be used.

The first culture unit 20 and the second culture unit 30 culture cells before forming the above-described sheet-shaped tissue or the like, and the third culture unit 40 cultures the sheet-shaped tissue or the like formed using cells cultured in the first and second culture units 20 and 30.

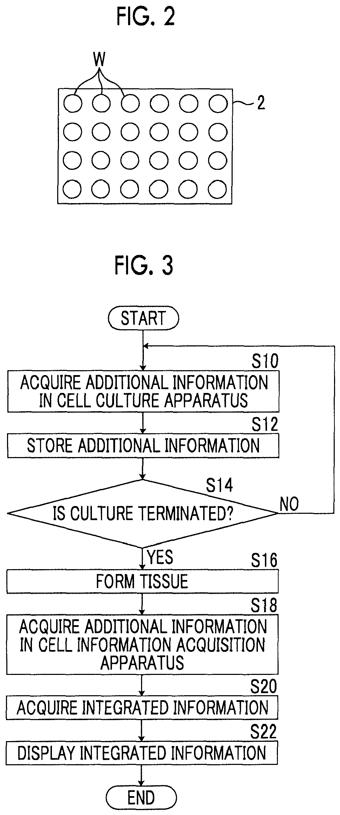

One or a plurality of culture vessels 2 in which the cells which are the culture targets are seeded in a culture medium is accommodated in the first culture unit 20 and the second culture unit 30. The culture vessel 2 used in this embodiment is a well plate provided with 24 wells of 4×6, as shown in Fig. 2. In each well W, a culture medium and one or a plurality of cell colonies which are culture targets are respectively accommodated, and the cell colonies are cultured in each well W. In this embodiment, each of the wells W corresponds to a cell culture unit in the invention.

Further, an additional information storage unit 3 is provided in each culture vessel 2, as shown in Fig. 1. The additional information storage unit 3 is configured by an information storage medium such as a radio frequency identifier (RFID) tag, a QR code (registered trademark) or a barcode, for example. Additional information for each well is stored in the additional information storage unit 3. Specific details of additional information assigned to each well W will be described in detail.

One or a plurality of culture plates 4 is accommodated in the third culture unit 40. In the culture plate 4, a plurality of cells cultured by the culture vessel 2 in the first and second culture units 20 and 30 is arranged in two dimensions or three dimensions, and a tissue formed of the plurality of cells is formed.

The arrangement of the plurality of cells in the culture plate 4 may be manually performed, or may be automatically performed. In a case where the arrangement of the plurality of cells is automatically performed, a configuration, in which cell colonies in each well W of the culture vessel 2 together with a culture medium are suctioned by a suction mechanism and the suctioned cell colonies are moved to a predetermined position on the culture plate 4 by a robot arm, may be used.

The controller 11 controls the entirety of the cell culture apparatus 10, and for example, controls culture conditions in the first to third culture units 20, 30, and 40. Specifically, the controller 11 controls culture conditions such as temperature, moisture, the type of a light source to be used, illuminance of the light source, oxygen concentration and carbon dioxide concentration in the first to third culture units 20, 30, and 40, and shaking conditions of a stage in which the culture vessel 2 is provided. As a configuration for adjusting the culture conditions, a known configuration may be used. Further, the respective culture conditions of the first to third culture units 20, 30, and 40 may be the same culture conditions, or may be different culture conditions.

In the cell culture apparatus 10 of this embodiment, the cell colonies cultured for each well W are arranged on the culture plate 4 in two dimensions or three dimensions to form a tissue of skin, organ or the like. Here, the tissue may be formed using cell colonies cultured by any culture unit among the first and second culture units 20 and 30, or may be formed using cell colonies cultured by a plurality of culture units. In this embodiment, the tissue corresponds to an integrative cell culture unit in the invention.

As shown in Fig. 1, an input device 12 and a display device 13 are connected to the cell culture apparatus 10.

The input device 12 includes an input device such as a mouse or a keyboard, and receives a setting input from a user. The input device 12 in this embodiment receives a setting input of the culture conditions in the first to third culture units 20 to 40 or a setting input of additional information stored in the additional information storage unit 3 of the culture vessel 2. The display device 13 includes a display device such as a liquid crystal display, and displays details of a setting input through the input device 12, for example. The display device 13 may be configured as a touch panel to further have the function of the input device 12.

The cell information acquisition apparatus 50 includes an additional information acquisition unit 51, an integrated information acquisition unit 52, and a controller 53. The cell information acquisition apparatus 50 is a device in which an embodiment of a cell information acquisition program of the invention is installed in a computer. The cell information acquisition apparatus 50 includes a central processing unit, a semiconductor memory, a hard disk, and the like, in which the embodiment of the cell information acquisition program is installed in the hard disc. Further, as the program is executed by a central processing unit provided in the controller 53, the additional information acquisition unit 51 and the integrated information acquisition unit 52 are operated.

The additional information acquisition unit 51 acquires additional information for each well W stored in the additional information storage unit 3 of each culture vessel 2. Hereinafter, a specific example of the additional information of this embodiment will be described.

The additional information of this embodiment may include well identification information for identifying each well W, information relating to a donor of cells cultured in each well W, information relating to a patient to whom a tissue formed from the cells cultured in each well W are provided, information relating to picking of the cells cultured in each well W, information relating to culture of the cells cultured in each well W, and the like, for example.

The information relating to the donor of the cells includes identification information of the donor, and information about age, gender, race, blood type, HLA (human Leukocyte Antigen) type, genetic type, disease, medical history, and the like.

The information relating to the patient is similar to the information relating to the donor.

The information relating to the picking of the cells includes information about a picking date in which cells used for culture are picked from a human body or the like, a picked portion, a picking amount, and a cell type indicating whether a picked cell is a stem cell or a cell other than the stem cell, for example.

The information relating to the culture of the cells includes information about a culture line (in this embodiment, the first or second culture units correspond to culture lines), a culture method, a culture period, a quality evaluation result, a validity evaluation result of a culture process, a cell image, and an evaluation result of a culture environment such as a culture medium or a scaffold, for example. The quality evaluation result includes cell colony form information, a well test result, a contamination test result, or the like.

The additional information includes at least one type of the above-described information, but is not limited to the above-described information, and may include any information which directly or indirectly relates to the cells cultured in each well W.

The integrated information acquisition unit 52 integrates, when forming a tissue using the plurality of cell colonies cultured in each well W as described above, additional information of the respective wells W to acquire integrated information relating to the tissue. The integration of the additional information refers to a process for causing a plurality of pieces of additional information to be collected and managed as one piece of information. As a method for collecting and managing the plurality of pieces of additional information as one piece of information, for example, a method for storing the information on a storage medium such as a single RFID tag, a method for storing the information on a predetermined storage medium together with header information or the like to be read as one piece of information, or a method for collectively storing the information in a single storage area of a predetermined storage medium.

The integrated information includes information obtained by maintaining the entirety of a plurality of pieces of additional information as it is and collect the information as one piece of information, information obtained by extracting partial information from a plurality of pieces of additional information and collecting the extracted information as one piece of information, information obtained by processing a plurality of pieces of additional information to be collected as one piece of information, or the like. The integrated information will be described in detail later.

The controller 53 controls the entirety of the cell information acquisition apparatus 50. Particularly, the controller 53 of this embodiment causes a display device 55 to display the integrated information acquired by the integrated information acquisition unit 52. Further, for example, in a case where the integrated information is to be stored in a single RFID tag, another server device, or the like, the controller 53 performs a process of storing the integrated information.

An input device 54 and the display device 55 are connected to the cell information acquisition apparatus 50. The input device 54 includes an input device such as a mouse or a keyboard, and receives a setting input from a user. Particularly, the input device 54 in this embodiment receives, when displaying integrated information, an instruction input therefor. The display device 55 includes a display device such as a liquid crystal display, and displays the integrated information as described above, for example. The display device 55 may be configured as a touch panel further having the function of the input device 54.

Next, an operation of the cell information management system according to this embodiment will be described with reference to a flowchart shown in Fig. 3.

First, in the first and second culture units 20 and 30 of the cell culture apparatus 10, cell colonies that form a desired tissue are cultured in each well W. Further, for example, before starting the culture or in the culture process, the above-described additional information of each well W is acquired by the controller 11 of the cell culture apparatus 10 (S10). The additional information of each well W acquired by the controller 11 is stored in the additional information storage unit 3 provided in each culture vessel 2 (S12).

The additional information may be acquired by a setting input using the input device 12 from a user, or may be acquired by receiving information output from another data server device in which additional information is stored, for example. Further, as image information about cells, an image captured by an imaging device such as a microscope (not shown) may be acquired.

In a case where culture of cells using the first and second culture units 20 and 30 is terminated (S14), cell colonies cultured by the plurality of wells W in a predetermined culture vessel 2 are arranged on the culture plate 4 of the third culture unit 40 in two dimensions or three dimensions to form a tissue, and then, culture of the tissue is performed (S16).

In addition, when the tissue is formed as described above, for example, according to an instruction input using the input device 54 from a user, the additional information of each well W in which the cells used in the tissue are cultured is read from each additional information storage unit 3, and is acquired by the additional information acquisition unit 51 of the cell information acquisition apparatus 50 (S18). The information about the well W in which the cells used in the tissue are cultured may be acquired by a setting input using the input device 12 from the user, or may be acquired, in a case where cell colonies are automatically arranged to form a tissue, based on control information about a robot arm for arranging the cell colonies, for example.

Then, the additional information for each well W acquired by the additional information acquisition unit 51 is output to the integrated information acquisition unit 52, and the integrated information acquisition unit 52 integrates the input additional information of the respective wells W to acquire integrated information relating to the tissue (S20).

The integrated information acquired by the integrated information acquisition unit 52 is output to the controller 53, and the controller 53 causes the display device 55 to display the integrated information according to an instruction input from the user, for example.

According to the cell information management system of this embodiment, when integrating cells which are respectively cultured by a plurality of wells W to form a tissue, since additional information assigned to the plurality of wells W is acquired and the additional information of the respective wells W is integrated to acquire the integrated information relating to the tissue, it is possible to secure traceability of the respective wells W with reference to the integrated information.

Next, a specific example of integrated information acquired by the integrated information acquisition unit 52 will be described.

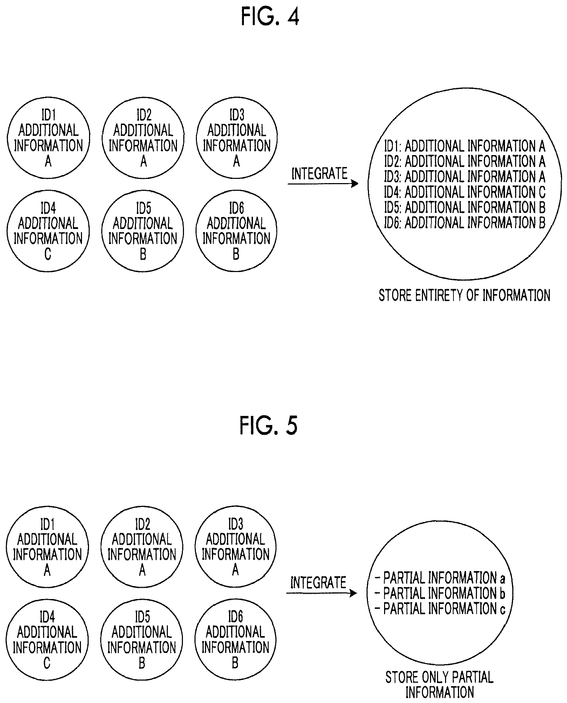

First, a case where the entirety of the additional information assigned to the respective wells W is maintained as it is to be integrated to form integrated information will be described. Fig. 4 is a schematic view illustrating this case. Six circles shown on the left side of Fig. 4 represent respective wells W used when forming a tissue, and show additional information of the respective wells W in the respective circles. Further, ID1 to ID6 shown in the circles of the respective wells W represent identification information for identifying the respective wells W.

Additional information A to C and the identification information ID1 to ID6 of the respective wells W are separately shown in the respective circles, but it is assumed that the identification information ID1 to ID6 is a type of additional information. Further, it is assumed that additional information having the same alphabet means that details of additional information other than identification information are the same. In other words, in the case of the example shown in Fig. 4, the additional information A of the identification information ID1 to ID3 has the same details in the additional information other than the identification information, and the additional information B of the identification information ID5 to ID6 has the same details in the additional information other than the identification information. A case where details of additional information other than identification information are the same and the identification information is different is a case where a plurality of different wells W cultured in a common culture unit is used to form a tissue, for example.

Further, integrated information relating to a tissue formed of cells of the respective wells W of ID1 to ID6 is shown in a large circle shown on the right side of Fig. 4.

Hereinafter, six circles shown on the left side of Figs. 5, and Figs. 7 to 12 and a large circle shown on the right side thereof have the same meanings as those in Fig. 4.

In the example shown in Fig. 4, in a state where the entirety of the additional information A to B of the respective wells W of ID 1 to ID6 is maintained as it is, the integrated information is acquired, as described above.

Here, in a case where the entire additional information is integrated to acquire integrated information as shown in Fig. 4, the additional information may be classified based on information about dates included in the respective pieces of additional information to acquire the integrated information. Specifically, in a case where cell picking dates are included in the respective pieces of additional information, for example, additional information of which the picking dates are the same may be grouped as a classification by comparing the picking dates of the respective pieces of additional information. Further, with respect to additional information that belongs to a common classification, a label or the like indicating the same group may be assigned for connection.

Further, in a case where operation dates when a user performs a certain operation for stimulating cells, in addition to the picking dates, are included in the respective pieces of additional information, additional information of which the operation dates are the same may be grouped as a classification. Further, in a case where culture starting dates are included in the respective pieces of additional information, additional information of which the culture starting dates are the same may be grouped as a classification. Information about dates used in classifying additional information, for example, information about dates such a picking dates, operation dates, or culture starting dates as described above is classified may be set in advance, or may be acquired by an arbitrary setting input using the input device 54 from a user.

Next, a case where partial information is extracted from additional information assigned to the respective cells W and the partial information is integrated to acquire integrated information will be described. Fig. 5 is a diagram schematically illustrating this case.

Specifically, for example, only the information about the culture conditions may be extracted from each of the respective pieces of additional information A to C to acquire partial information a to c and the partial information a to c may be integrated to acquire integrated information, or only the information relating to the donor may be extracted therefrom to acquire the partial information a to c and the partial information a to c may be integrated to acquire integrated information. Further, the partial information is not limited to the above-described information about the culture conditions or the information about the donor, and for example, information relating to a specific date which is set in advance, information about a quality evaluation result, or information about a history of the quality evaluation result, or the like may be used as the partial information.

The information relating to the specific date includes information about an operation performed with respect to cells on the specific date, information about culture conditions or a culture environment at the specific date, or the like, for example. The specific date may be set in advance, or may be acquired by an arbitrary setting input using the input device 54 from a user.

Details of the partial information to be extracted from the respective pieces of additional information may be set in advance, or may be acquired by an arbitrary setting input using the input device 54 from a user.

Further, since necessary information is different according to a desired tissue, details of the partial information may be determined according to the type of the tissue. Specifically, for example, in a case where the tissue is a cartilage, since rejection does not easily occur, HLA-type information is not necessary, but in a case where the tissue is not the cartilage, since the rejection easily occurs, HLA-type information is necessary. Accordingly, in a case where the desired tissue is the cartilage, partial information in which the HLA-type information is not included may be extracted, and in a case where the desired tissue is not the cartilage, partial information in which the HLA-type information is included may be extracted.

Further, in a case where the desired tissue is blood, since a blood type of a donor or a patient is important and an evaluation result of the number of remaining stem cells is not necessary, information about the blood type of the donor or the patient may be included in partial information, and the evaluation result of the number of remaining stem cells may not be included therein.

In addition, in a case where the desired tissue is myocardium, if undifferentiated stem cells remain, since the undifferentiated stem cells are differentiated after transplanting to cause tumor or cancer, the evaluation result of the number of remaining stem cells may be necessarily included in partial information.

Furthermore, in a case where the desired tissue is skin, since there is a case where information about race is necessary with respect to a skin color, the information about the race may be necessarily included in partial information.

In a case where information acquired a plurality of times in a temporal direction is included in the additional information about the respective wells W, only partial information among the information acquired the plurality of times may be extracted to acquire integrated information. Specifically, for example, in a case where the desired tissue is myocardium or skin, it is sufficient if stem cells finally do not remain in the tissue. Thus, for example, in a case where results obtained by evaluating the number of stem cells a plurality of times in a temporal direction is included in the additional information about the respective wells W, only a final evaluation result may be extracted and other evaluation results may be deleted to acquire integrated information.

In a case where the desired tissue is a tissue formed of stem cells through iPS cells, information about a quality evaluation result at an initial culture stage, information about a culture line, or information relating to a culture method is important. Accordingly, in a case where a plurality of pieces of information acquired in a temporal direction is included in the additional information about the respective wells W with respect to the above-described important information, information acquired at the initial culture stage may be extracted from the plurality of pieces of information and other information may be deleted to acquire integrated information. The period of the initial culture stage may be set in advance, or may be acquired by an arbitrary setting input using the input device 54 from a user.

Further, in a case where the desired tissue is a nerve, there is a case where the quality of the nerve is evaluated by an axon growth speed in a middle stage which is an initial differentiation stage. Accordingly, in a case where results obtained by evaluating the axon growth speed a plurality of times in a temporal direction is included in the additional information about the respective wells W, only an evaluation result in the middle stage may be extracted and other evaluation results may be deleted to acquire integrated information. The period of the middle stage may be set in advance, or may be acquired by an arbitrary setting input using the input device 54 from a user.

In addition, as described above, in a case where the desired tissue is myocardium and skin, a tissue formed through stem cells from iPS cells, or a nerve, at an initial culture stage, a middle culture stage, and a later culture stage, information as indicated in the following table is importantly evaluated. Accordingly, in a case where an evaluation result of the information as shown in the following table is included in the additional information about the respective wells W, the evaluation result may be extracted and other evaluation results or information may be deleted to acquire integrated information.

**[Table 1]**

| Type of tissue | Initial stage | Middle stage | Later stage |
|---|---|---|---|
| Myocardium and skin (based on stem cells) | Stem cells or density, presence or absence of contamination, ... | Desired cell form evaluation information, ... | The number of remaining stem cells, the number of desired cells, or density, ... |
| Tissue formed from cells based on iPS cells | Colony form information, the number of iPS cells or density, ... | Form evaluation information about cells differentiated from iPS cells | The number of remaining stem cells, the number of desired cells, or density, ... |
| Nerve | The number of stem cells or density, proliferation speed, ... | Axon length or axon extension speed, ... | The number of stem cells, ... |

Further, in the above table, information at the later stage is most important with respect to the myocardium and skin, information at the initial stage is most important with respect to the tissue formed through the stem cells from the iPS cells, and information at the middle stage is most important with respect to the nerve. Accordingly, for example, in a case where the desired tissue is myocardium or skin, the number of evaluation results of the information at the later stage may be relatively increased and the number of evaluation results of the information at the initial stage and the middle stage may be relatively decreased and extracted to acquire integrated information.

Further, in a case where the desired tissue is a tissue formed through the stem cells from the iPS cells, the number of evaluation results of the information at the initial stage may be relatively increased and the number of evaluation results of the information at the middle stage and the later stage may be relatively decreased and extracted to acquire integrated information. Further, in a case where the desired tissue is nerve, the number of evaluation results of the information at the middle stage may be relatively increased and the number of evaluation results of the information at the initial stage and the later stage may be relatively decreased and extracted to acquire integrated information.

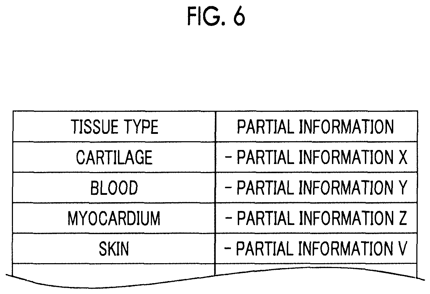

As described above, as a method for specifying details of partial information based on a desired tissue, for example, as shown in Fig. 6, a method for setting in advance a table in which the type of the tissue and details of the partial information based on the type are associated with each other, and performing a setting input of the desired tissue using the input device 54 from a user to specify the details of the partial information based on the tissue may be used.

Further, in the above description, partial information to be integrated is specified according to a desired tissue, but partial information to be included in integrated information is changed according to whether the desired tissue is used for regenerative medicine or for drug discovery. Accordingly, the partial information may be specified according to whether the desired tissue is used for regenerative medicine or for drug discovery. A table in which the usage of the tissue and the partial information to be integrated are associated with each other may be set in advance.

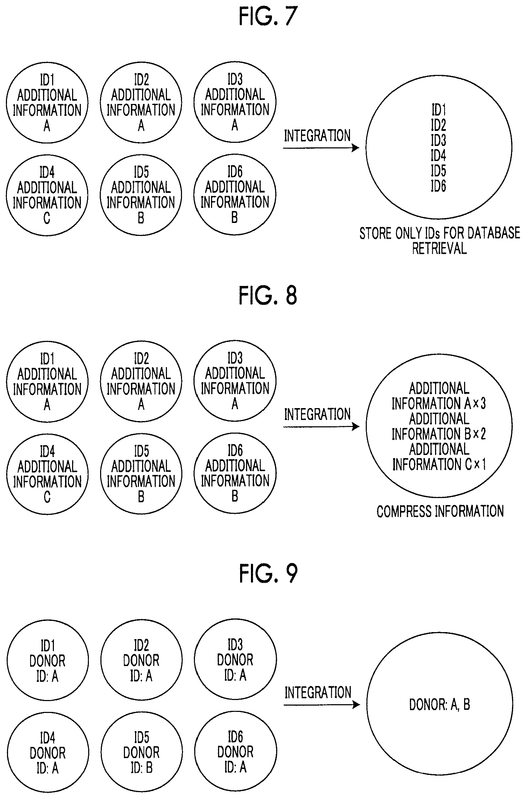

In addition, for example, in a case where the additional information about the respective wells W is stored and managed in a data server device or the like other than the additional information storage unit 3, retrieval indexes used for retrieving and specifying the additional information about the respective wells W may be extracted from the data server device as partial information, and the retrieval indexes may be integrated to acquire integrated information. Fig. 7 is a diagram schematically illustrating this case. As the above-described retrieval indexes, for example, as shown in Fig. 7, identification information ID1 to ID6 of the respective wells W are given. The retrieval indexes are not limited to the identification information of the respective wells W, and may be any other information capable of specifying the respective wells W.

Further, in a case where the retrieval indexes are integrated to acquire the integrated information as described above, in a case where it is considered that a user wants to check additional information about a predetermined well W, the user inputs identification information about the well W using the input device 54. Then, the controller 53 may retrieve the data server device based on the identification information, may acquire additional information based on the identification information, and may cause the display device 55 to display the additional information.

By extracting and integrating the partial information as described above, it is possible to reduce the memory capacity of the integrated information, and to leave only necessary information.

Next, a case where additional information assigned to the respective wells W is processed to acquire integrated information will be described. Fig. 8 is a diagram schematically illustrating a case where a compression process is performed as a processing process.

Here, as a method of a compression process of respective pieces of additional information, for example, in a case where there is a plurality of pieces of additional information in which details other than identification information of the respective wells W are the same, a method for leaving only one piece of additional information among the plurality of pieces of additional information and compressing the remaining additional information may be used. Further, in this case, it is preferable that the number of the pieces of compressed additional information before compression is assigned, and thus, it is possible to check the ratio of the number of wells W to which the compressed additional information is assigned. In the example of Fig. 8, with respect to additional information A, three pieces of additional information A are compressed into one piece of additional information A, and "3" which is the number of pieces of additional information A before compression is assigned to the additional information A after the compression. In addition, with respect to additional information B, two pieces of additional information B are compressed into one piece of additional information B, and "2" which is the number of additional information B before compression is assigned to the additional information B after the compression. With respect to additional information C, since only one piece of additional information is present from the beginning, "1" is assigned to the additional information C.

As described above, the additional information to be compressed includes information about a culture line, information relating to culture conditions, or the like, for example.

Further, in the compression method, three pieces of additional information A to C remains as they are, but for example, in a case where similar additional information is present, the similar additional information may be considered as one piece of representative additional information for compression. Specifically, as shown in Fig. 8, in a case where three pieces of additional information A, two pieces of additional information B, and one piece of additional information C are compressed, for example, in a case where the additional information B and the additional information C are similar to each other, the additional information C may be considered as the additional information B for compression. In this case, the number of pieces of additional information C is also added to the number of pieces of additional information B after compression. In other words, the number of pieces of additional information B after compression becomes "3".

As a method for determining similar additional information, for example, in a case where additional information is information relating to culture, in a case where there is a plurality of pieces of additional information in which only information which is not so important is different and other information is the same, among the information relating to the culture, it may be determined that the plurality of pieces of additional information is similar to each other. The information which is not important may be set in advance, or may be determined by an arbitrary setting input using the input device 54 from a user.

The compression method is not limited to the above-described method, and for example, a method for maintaining information having a high confirmation priority for a user, among information included in the respective pieces of additional information A to C, and collecting information having a low priority as one piece of information for compression may be used. The information to be remained as it is without being compressed and the information to be compressed, among the information included in the additional information, may be set in advance, or may be determined by an arbitrary setting input using the input device 54 from a user.

Further, for example, as shown in Fig. 9, in a case where donor identification information is included in additional information of the respective wells W and the same donor identification information is present, the same donor information may be connected for compression. In the case of an example shown in Fig. 9, since five pieces of donor identification information A included in the additional information of the respective wells W are the same, the five pieces of donor identification information A are connected to obtain one piece of donor identification information A. In other words, only two pieces of donor information, that is, A and B are included in the integrated information as the donor identification information.

As described above, by compressing the additional information of the respective wells W to acquire integrated information, it is possible to reduce a storage capacity for the integrated information, and to leave only necessary information.

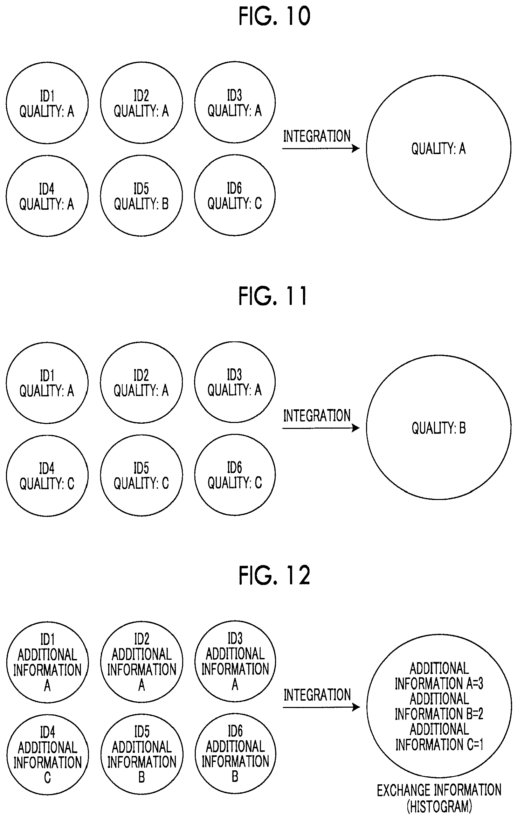

Next, a case where a process of converting respective pieces of additional information into secondary information is performed as a processing process for additional information assigned to the respective wells W to acquire integrated information will be described. Figs. 10 and 11 are diagrams schematically illustrating this case.

Specifically, for example, in a case where information about quality evaluation results is included in the additional information assigned to the respective wells W, information about a plurality of different quality evaluation results may be converted into information about one quality evaluation result to acquire integrated information. For example, as shown in Fig. 10, quality evaluation results A to C assigned to the respective wells W may be converted into one quality evaluation result A having the highest frequency to acquire integrated information. Further, as shown in Fig. 11, the quality evaluation results A and C assigned to the respective wells W may be converted into the quality evaluation result B which is an average quality evaluation result to acquire integrated information. Here, for example, it is assumed that, among the quality evaluation results A to C, the quality evaluation result A is the best evaluation result, the quality evaluation result C is the worst evaluation result, and the quality evaluation result B is an evaluation result between the quality evaluation result A and the quality evaluation result C.

Further, the above-described conversion methods are not limiting, and for example, the quality evaluation results A to C may be converted into the quality evaluation result C which is the worst evaluation result among the quality evaluation results A to C to acquire integrated information.

As described above, by converting information about a plurality of different quality evaluation results into information about one quality evaluation result to acquire integrated information, it is possible to reduce a storage capacity for the integrated information, and to acquire information necessary for tissue evaluation.

In addition, in a case where information about quality evaluation results is included in the additional information assigned to the respective wells W and the quality evaluation results are overlapped, similar to the above-described case of the example shown in Fig. 9, the overlapped quality evaluation results may be connected to each other for compression. Specifically, in a case where a quality evaluation result of each well A is an example as shown in Fig. 10, since four pieces of information A about the quality evaluation results are included in the additional information of the respective wells W and are overlapped, the overlapped information A may be connected to each other to be compressed as information A about one quality evaluation result.

In a case where information about quality evaluation results is included in the additional information assigned to the respective wells W, as described above, whether to convert the quality evaluation results into a quality evaluation result having the highest frequency to acquire integrated information, to convert the quality evaluation results into an average quality evaluation result to acquire integrated information, or to connect overlapped quality evaluation results to be compressed as one quality evaluation result, may be selected according to a desired tissue. Specifically, for example, a method for setting in advance a table in which the type of a tissue and a method for acquiring integrated information according to the type of the tissue are associated with each other and performing a setting input a desired tissue using the input device 54 from a user to acquire integrated information based on the tissue may be selected.

For example, in a case where it is expected that a quality variation according to a raising method is large and a distribution of quality evaluation information is complicated, as in stem cells, it is preferable to convert quality evaluation results into an average quality evaluation result to acquire integrated information. Further, in a case where a quality variation due to a growing method is small and a quality evaluation information distribution is unimodal, it is preferable to convert quality evaluation results into a quality evaluation result having the highest frequency to acquire integrated information.

In addition, the additional information assigned to the respective wells W may be converted into information capable of being represented as a visual graph. Fig. 12 is a diagram schematically illustrating this case. The example shown in Fig. 12 corresponds to an example in which additional information A to C is converted into information capable of being represented as a visual histogram, in which information about the frequency is added to the respective pieces of additional information A to C to acquire integrated information. In this case, for example, the controller 53 may cause the display device 55 to display histograms of the additional information A to C based on the integrated information.

Furthermore, the additional information assigned to the respective wells W may be converted into information relating to a temporal change to acquire integrated information. Specifically, for example, in a case where information about a plurality of quality evaluation results acquired in a temporal direction is included in the additional information assigned to the respective wells W, the information about the quality evaluation results may be converted into information relating to a temporal change of the quality evaluation results or change point information such as a point of time at which a quality evaluation result exceeds a threshold value to acquire integrated information.

As described above, by converting respective pieces of additional information into information capable of being represented as a visual graph or information relating to a temporal change, it is possible to provide new information relating to a tissue to a user.

In the above-described embodiment, a configuration in which additional information assigned to the respective wells W is integrated to acquire integrated information is shown, but a configuration in which information about tissues formed using cells of the respective wells W is further included in integrated information may be used. The information about tissues may include information relating to tissue processing, information relating to qualities of final processed products, or the like. Specifically, the information relating to tissue processing may include information about an intermediate processing line, information about an intermediate processing method (planar configuration, stereoscopic configuration, or the like), information about a contamination test result, information about an intermediate process validity evaluation result, and the like. Further, the information about qualities of final processed products may include information about the number of cells in a tissue, information about the number of surviving cells, information about a stem cell remaining check result, information about a potency test result, information about a cell feature test result, and information about a germless mycoplasma prime test result, and the like.

As described above, by causing integrative tissue information to be included in integrated information, it is possible to achieve enhancement of traceability as a final product.

Further, in the above-described embodiment, a configuration in which each well W corresponds to a cell culture unit of the invention is shown as described above, but since a cell culture unit is a unit for culturing cells, it is not essential that the cell culture unit is a well unit as described in this embodiment. That is, the cell culture unit may be an individual cell unit, a cell colony unit, a tissue unit, a culture vessel unit, or the like.

Furthermore, in the above-described embodiment, a configuration in which a tissue formed of cells cultured by the respective wells W corresponds to an integrative cell culture unit of the invention is shown. However, it is sufficient if an integrative cell culture unit is a unit larger than a cell culture unit. Thus, for example, in a case where the cell culture unit is a cell, the integrative cell culture unit may be a cell colony, a well, a tissue, or a culture vessel. Further, in a case where the cell culture unit is a cell colony, the integrative cell culture unit may be a well, a tissue, or a culture vessel. In addition, in a case where the cell culture unit is a well, the integrative cell culture unit may be a tissue or a culture vessel.

### Explanation of References

1: cell information management system
2: culture vessel
3: additional information storage unit
4: culture plate
10: cell culture apparatus
11: controller
12: input device
13: display device
20, 30, 40: first to third culture units
50: cell information acquisition apparatus
51: additional information acquisition unit
52: integrated information acquisition unit
53: controller
54: input device
55: display device

## Claims

1. A management system (1) including a cell culture apparatus (10) and a cell information acquisition apparatus (50), the cell information acquisition apparatus comprising:
an additional information acquisition unit (51) adapted to acquire additional information assigned to a plurality of cell culture units (20, 30) each comprising a culture vessel (2) and an additional information storage unit (3), the additional information including information relating to a donor of cells cultured in a culture vessel (2) including identification information of the donor, information about age, gender, race, blood type, human Leukocyte Antigen type, genetic type, disease, information relating to the culture of the cells, medical history, and information relating to picking of the cells;
an integrated information acquisition unit (52) adapted to acquire, when integrating cells respectively cultured in the plurality of cell culture units (20, 30) to create an integrative cell culture unit (40) in form of a tissue, integrated information relating to the integrative cell culture unit (40) by integrating the additional information of each cell culture unit (20, 30) by a process for causing a plurality of pieces of additional information to be collected and managed as one piece of information,
wherein the integrated information acquisition unit (52) is adapted to acquire the integrated information by extracting partial information from the additional information assigned to the respective cell culture units (20, 30) and integrating the partial information,
wherein the information relating to the picking of the cells includes at least information about a picking date, a picked portion, a picking amount and a cell type, and the information of the culture of the cells includes at least information about a culture line, a culture method, a culture period, a quality evaluation result, a validity valuation result of a culture process, a cell image and an evaluation result of a culture environment, and
wherein the additional information is collected and managed as one piece of information by
- storing the information on a storage medium such as a single RFID tag provided in each culture vessel, or by
- storing the information on a predetermined storage medium together with header information or the like to be read as one piece of information, or by
- storing the information on a single storage area of a predetermined storage medium.

2. The system (1) according to claim 1,
wherein the integrated information acquisition unit (52) acquires the integrated information by integrating the entirety of the additional information assigned to the respective cell culture units (20, 30).

3. The system (1) according to claim 2,
wherein the integrated information acquisition unit (52) acquires the integrated information by classifying the additional information based on dates.

4. The system (1) according to claim 1,
wherein the partial information includes a culture condition of each cell culture unit (20, 30).

5. The system (1) according to any one of claims 1 to 4,
wherein the partial information includes a retrieval index used for retrieving the additional information of each cell culture unit.

6. The system (1) according to any one of claims 1 to 5,
wherein the partial information includes information relating to a specific date that is set in advance.

7. The system (1) according to claim 1,
wherein the integrated information acquisition unit (52) acquires the integrated information by processing the additional information assigned to the respective cell culture units (20, 30).

8. The system (1) according to claim 7,
wherein the integrated information acquisition unit (52) acquires the integrated information by compressing the additional information assigned to the respective cell culture units (20, 30).

9. The system (1) according to claim 8,
wherein the integrated information acquisition unit (52) acquires the integrated information by converting the additional information assigned to the respective cell culture units (20, 30) into secondary information based on the additional information.

10. The system (1) according to claim 9,
wherein the secondary information is quality information about the integrative cell culture unit (40).

11. The system (1) according to claim 9,
wherein the secondary information is information capable of representing the additional information as a visual graph.

12. The system (1) according to claim 9,
wherein the secondary information is information relating to a temporal change.

13. The system (1) according to any one of claims 1 to 12,
wherein the integrated information acquisition unit (52) causes information acquired with respect to the integrative cell culture unit (40) to be included in the integrated information.

14. A cell information acquisition method executed by a system (1) according to any one of claims 1 to 13, comprising:
acquiring (S10) additional information assigned to a plurality of cell culture units (20, 30) comprising a culture vessel (2) and an additional information storage unit (3), the additional information including information relating to a donor of cells including identification information of the donor, and information about age, gender, race, blood type, human Leukocyte Antigen type, genetic type, disease, information relating to the culture of the cells, medical history, and information relating to picking of the cells,
acquiring (S20), when integrating cells respectively cultured in the plurality of cell culture units (20, 30) to create an integrative cell culture unit (40) in form of a tissue, integrated information relating to the integrative cell culture unit (40) by integrating the additional information of the respective cell culture units (20, 30) by collecting and managing a plurality of pieces of additional information as one piece of information,
acquiring (S20) the integrated information by extracting partial information from the additional information assigned to the respective cell culture units (20, 30) and integrating the partial information,
wherein the information relating to the picking of the cells includes at least information about a picking date, a picked portion, a picking amount and a cell type, and the information of the culture of the cells includes at least information about a culture line, a culture method, a culture period, a quality evaluation result, a validity valuation result of a culture process, a cell image and an evaluation result of a culture environment, and
wherein the additional information is collected and managed as one piece of information by
- storing the information on a storage medium such as a single RFID tag provided in each culture vessel, or by
- storing the information on a predetermined storage medium together with header information or the like to be read as one piece of information, or by
- storing the information on a single storage area of a predetermined storage medium.

15. A cell information acquisition program that, when executed on a computer (53) of a system (1) according to any one of claims 1 to 13, causes the computer (53) to conduct the method according to claim 14.

## Patentansprüche

1. Verwaltungssystem (1), enthaltend eine Zellkulturvorrichtung (10) und eine Zellinformations-Erfassungsvorrichtung (50), wobei letztere aufweist:
eine Zusatzinformations-Erfassungseinheit (51), ausgebildet zum Erfassen von Zusatzinformation, die mehreren Zellkultureinheiten (20, 30) zugeordnet ist, die jeweils ein Kulturgefäß (2) und eine Zusatzinformations-Speichereinheit (3) enthalten, die Zusatzinformation Information über einen Spender von in einem Kulturgefäß (2) gezüchteten Zellen mit Spender-Kennungsinformation, Information über Alter, Geschlecht, Rasse, Bluttyp, menschlichen Leukozyten-Antigen-Typ, genetischen Typ, Krankheit, Information über die Züchtung der Zellen, medizinische Vorgeschichte und Information über die Entnahme der Zellen enthält;
eine Integrierte-Informations-Erfassungseinheit (52), ausgebildet, um, wenn in mehreren Zellkultureinheiten (20,30) gezüchtete Impfzellen integriert werden zum Erzeugen einer integrierten Zellkultureinheit (40) in Form eines Gewebes, integrierte Information über die integrierte Zellkultureinheit (40) zu erfassen, indem die Zusatzinformation jeder Zellkultureinheit (20, 30) integriert wird durch einen Prozess des Veranlassens, mehrere Stücke von Zusatzinformation als ein Informationsstück zu sammeln und zu verwalten,
wobei die Integrierte-Informations-Erfassungseinheit (52) ausgebildet ist zum Erfassen der integrierten Information durch Extrahieren von Teilinformation aus der den jeweiligen Zellkultureinheiten (20, 30) zugeordneter Zusatzinformation und Integrieren der Teilinformation,
wobei die Information über die Entnahme der Zellen mindestens Information über ein Entnahmedatum, ein Entnahmeteil, eine Entnahmemenge und einen Zellentyp enthält, und die Information der Züchtung der Zellen mindestens Information über eine Züchtungslinie, ein Züchtungsverfahren, eine Züchtungszeitspanne, ein qualitatives Bewertungsergebnis, ein Gültigkeitsbewertungsergebnis eines Züchtungsprozesses, ein Zellenbild und ein Bewertungsergebnis einer Züchtungsumgebung enthält, und,
wobei die Zusatzinformation als ein Informationsstück gesammelt und verwaltet wird, indem
- die Information in einem Speichermedium wie zum Beispiel einem einzelnen RFID-Etikett gespeichert wird, das in jedem Kulturgefäß enthalten ist, oder
- die Information zusammen mit Vorsatzinformation oder dergleichen zum Auslesen als ein Informationsstück auf einem vorbestimmten Speichermedium gespeichert wird, oder
- die Information in einem einzelnen Speicherbereich eines vorbestimmten Speichermediums gespeichert wird.

2. System (1) nach Anspruch 1,
bei dem die Integrierte-Informations-Erfassungseinheit (52) die integrierte Information dadurch erfasst, dass sie die Gesamtheit der den jeweiligen Zellkultureinheiten (20, 30) zugeordneten Zusatzinformation integriert.

3. System (1) nach Anspruch 2,
bei dem die Integrierte-Informations-Erfassungseinheit die integrierte Information durch Klassifizieren der Zusatzinformation basierend auf Datumsangaben klassifiziert.

4. System (1) nach Anspruch 1,
bei dem die Teilinformation eine Züchtungsbedingung für jede der Zellkultureinheiten (20, 30) enthält.

5. System (1) nach einem der Ansprüche 1 bis 4,
bei dem die Teilinformation einen Wiederauffindindex enthält, verwendet zum Wiederauffinden der Zusatzinformation jeder Zellkultureinheit.

6. System (1) nach einem der Ansprüche 1 bis 5,
bei dem die Teilinformation Information über ein vorab eingestelltes spezifisches Datum enthält.

7. System (1) nach Anspruch 1,
bei dem die Integrierte-Informations-Erfassungseinheit (52) die integrierte Information erfasst durch Verarbeiten der Zusatzinformation den jeweiligen Zellkultureinheiten (20, 30) zugeordnet ist.

8. System (1) nach Anspruch 7,
bei dem die Integrierte-Informations-Erfassungseinheit (52) die integrierte Information erfasst durch Komprimieren der den jeweiligen Zellkultureinheiten (20, 30) zugeordnet sind Zusatzinformation.

9. System (1) nach Anspruch 8,
bei dem die Integrierte-Informations-Erfassungseinheit (52) die integrierte Information erfasst durch Umwandeln der den jeweiligen Zellkultureinheiten (20, 30) zugeordneten Zusatzinformation in auf der Zusatzinformation basierende Sekundärinformation.

10. System (1) nach Anspruch 9,
bei dem die Sekundärinformation Qualitätsinformation über die integrierte Zellkultureinheit (40) ist.

11. System (1) nach Anspruch 9,
bei dem die Sekundärinformation Information ist, die die Zusatzinformation als visuellen Graph repräsentieren kann.

12. System (1) nach Anspruch 9,
bei dem die Sekundärinformation Information über eine zeitliche Änderung ist.

13. System (1) nach einem der Ansprüche 1 bis 12,
bei dem die Integrierte-Informations-Erfassungseinheit (52) Information, die bezüglich der integrierten Zellkultureinheit (40) erfasste Information dazu bringt, in der integrierten Information enthalten zu sein.

14. Zellinformations-Erfassungsverfahren, ausgeführt von einem System (1) nach einem der Ansprüche 1 bis 13, umfassend:
Erfassen (S10) von Zusatzinformation, die mehreren Zellkultureinheiten (20, 30) zugeordnet ist, die ein einschließlich Kennungsinformation des Spenders und Information über Alter, Geschlecht, Rasse, Bluttyp, menschlichen Leukozyten-Antigen-Typ, genetischen Typ, Krankheit, Information über die Kultur der Zellen, medizinische Vorgeschichte und Information bezüglich der Entnahme der Zellen enthält;
wenn die jeweilige in mehreren Zellkultureinheiten (20,30) Kultureinheiten (20,30) gezüchtete Zellen integriert werden, um eine integrierte Zellkultureinheit (40) in Form eines Gewebes zu erzeugen, integrierte Information über die integrierte Zellkultureinheit (40) erfasst wird durch Integrieren der Zusatzinformation der jeweiligen Zellkultureinheiten (20, 30) durch Sammeln und Verwalten mehrerer Stücke von Zusatzinformation als ein Informationsstück,
Erfassen (S20) der integrierten Information durch Extrahieren von Teilinformation aus der den jeweiligen Zellkultureinheiten (20, 30) zugeordneter Zusatzinformation und Integrieren der Teilinformation,
wobei die Information über die Entnahme der Zellen mindestens Information über ein Entnahmedatum, ein Entnahmeteil, eine Entnahmemenge und einen Zellentyp enthält, und Information der Kultur der Zellen mindestens Information über eine Züchtungslinie, ein Züchtungsverfahren, eine Züchtungszeitspanne, ein qualitätsbewertungsergebnis, ein Gültigkeitsbewertungsergebnis eines Züchtungsprozesses, ein Zellenbild und ein Bewertungsergebnis einer Züchtungsumgebung enthält, und,
wobei die Zusatzinformation als ein Informationsstück gesammelt und verwaltet wird durch
- die Information in einem Speichermedium wie zum Beispiel einem einzelnen RFID-Etikett gespeichert wird, das in jedem Kulturgefäß enthalten ist, oder
- die Information zusammen mit Vorsatzinformation oder dergleichen zum Auslesen als ein Informationsstück auf einem vorbestimmten Speichermedium gespeichert wird, oder
- die Information in einem einzelnen Speicherbereich eines vorbestimmten Speichermediums gespeichert wird.

15. Zellinformations-Erfassungsprogramm, das bei Ausführung auf einem Computer (53) eines Systems (1) nach einem der Ansprüche 1 bis 13 den Computer (53) veranlasst, das Verfahren nach Anspruch 14 auszuführen.

## Revendications

1. Système de gestion (1) incluant un appareil de culture cellulaire (10) et un appareil d'acquisition d'informations cellulaires (50), l'appareil d'acquisition d'informations cellulaires comprenant :
une unité d'acquisition d'informations supplémentaires (51), apte à acquérir des informations supplémentaires attribuées à une pluralité d'unités de culture cellulaire (20, 30), chacune comprenant un récipient de culture (2) et une unité de stockage d'informations supplémentaires (3), les informations supplémentaires incluant des informations concernant un donneur de cellules cultivées dans un récipient de culture (2) incluant des informations d'identification du donneur, des informations sur l'âge, le sexe, la race, le groupe sanguin, le type d'antigène leucocytaire humain, le type génétique, une maladie, des informations concernant la cuture des cellules, les antécédents médicaux, et des informations concernant le prélèvement des cellules ;
une unité d'acquisition d'informations intégrées (52), apte à acquérir, lors de l'intégration de cellules cultivées respectivement dans la pluralité d'unités de culture cellulaire (20, 30) pour créer une unité de culture cellulaire intégrative (40) sous la forme d'un tissu, des informations intégrées concernant l'unité de culture cellulaire intégrative (40) en intégrant les informations supplémentaires de chaque unité de culture cellulaire (20, 30) par un processus faisant en sorte qu'une pluralité d'éléments d'informations supplémentaires soient collectés et gérés sous la forme d'une seule information,
dans lequel l'unité d'acquisition d'informations intégrées (52) est apte à acquérir les informations intégrées en extrayant des informations partielles des informations supplémentaires attribuées aux unités de culture cellulaire respectives (20, 30) et en intégrant les informations partielles ;
dans lequel les informations concernant le prélèvement des cellules incluent au moins des informations concernant une date de prélèvement, une portion prélevée, une quantité de prélèvement, et un type de cellule, et les informations de la culture des cellules incluent au moins des informations concernant une ligne de culture, une méthode de culture, une période de culture, un résultat d'évaluation de la qualité, un résultat d'évaluation de la validité d'un processus de culture, une image de cellule, et un résultat d'évaluation d'un environnement cellulaire, et
dans lequel les informations supplémentaires sont collectées et gérées sous la forme d'une seule information en
- stockant les informations sur un support de stockage tel qu'une étiquette d'identification par radiofréquence (RFID, radiofrequeny identification tag) prévue dans chaque récipient de culture, ou en
- stockant les informations sur un support de stockage prédéterminé conjointement avec des informations d'en-tête ou informations similaires devant être lues sous la forme d'une seule information, ou en
- stockant les informations sur une zone de stockage unique d'un support de stockage prédéterminé.

2. Système (1) selon la revendication 1,
dans lequel l'unité d'acquisition d'informations intégrées (52) acquiert les informations intégrées en intégrant la totalité des informations supplémentaires attribuées aux unités de culture cellulaire respectives (20, 30).

3. Système (1) selon la revendication 2,
dans lequel l'unité d'acquisition d'informations intégrées (52) acquiert les informations intégrées en classant les informations supplémentaires sur la base de dates.

4. Système (1) selon la revendication 1,
dans lequel les informations partielles incluent une condition de culture de chaque unité de culture cellulaire (20, 30).

5. Système (1) selon l'une quelconque des revendications 1 à 4,
dans lequel les informations partielles incluent un index de recherche utilisé pour rechercher les informations supplémentaires de chaque unité de culture cellulaire.

6. Système (1) selon l'une quelconque des revendications 1 à 5,
dans lequel les informations partielles incluent des informations concernant une date spécifique, laquelle est réglée à l'avance.

7. Système (1) selon la revendication 1,
dans lequel l'unité d'acquisition d'informations intégrées (52) acquiert les informations intégrées en traitant les informations supplémentaires attribuées aux unités de culture cellulaire respectives (20, 30).

8. Système (1) selon la revendication 7,
dans lequel l'unité d'acquisition d'informations intégrées (52) acquiert les informations intégrées par compression des informations supplémentaires attribuées aux unités de culture cellulaire respectives (20,30).

9. Système (1) selon la revendication 8,
dans lequel l'unité d'acquisition d'informations intégrées (52) acquiert les informations intégrées en convertissant les informations supplémentaires attribuées aux unités de culture cellulaire respectives (20,30) en informations secondaires sur la base des informations supplémentaires.

10. Système (1) selon la revendication 9,
dans lequel les informations secondaires sont des informations sur la qualité concernant l'unité de culture cellulaire intégrative (40).

11. Système (1) selon la revendication 9,
dans lequel les informations secondaires sont des informations en mesure de représenter les informations supplémentaires sous la forme d'un graphe visuel.

12. Système (1) selon la revendication 9,
dans lequel les informations secondaires sont des informations concernant un changement temporel.

13. Système (1) selon l'une quelconque des revendications 1 à 12,
dans lequel l'unité d'acquisition d'informations intégrées (52) fait en sorte que les informations acquises par rapport à l'unité de culture cellulaire intégrative (40) soient incluses dans les informations intégrées.

14. Procédé d'acquisition d'informations cellulaires exécuté par un système (1) selon l'une quelconque des revendications 1 à 13, comprenant les étapes consistant à :
acquérir (S10) des informations supplémentaires attribuées à une pluralité d'unités de culture cellulaire (20, 30), comprenant un récipient de culture (2) et une unité de stockage d'informations supplémentaires (3), les informations supplémentaires incluant des informations concernant un donneur de cellules incluant des informations d'identification du donneur, et des informations sur l'âge, le sexe, la race, le groupe sanguin, le type d'antigène leucocytaire humain, le type génétique, une maladie, des informations concernant la cuture des cellules, les antécédents médicaux, et des informations concernant le prélèvement des cellules ;
acquérir (S20), lors de l'intégration de cellules cultivées respectivement dans la pluralité d'unités de culture cellulaire (20, 30) pour créer une unité de culture cellulaire intégrative (40) sous la forme d'un tissu, des informations intégrées concernant l'unité de culture cellulaire intégrative (40) en intégrant les informations supplémentaires des unités de culture cellulaire respectives (20, 30), et en collectant et gérant une pluralité d'éléments d'informations supplémentaires sous la forme d'une seule information ;
acquérir (S20) les informations intégrées en extrayant des informations partielles des informations supplémentaires attribuées aux unités de culture cellulaire respectives (20, 30), et en intégrant les informations partielles ;
dans lequel les informations concernant le prélèvement des cellules incluent au moins des informations concernant une date de prélèvement, une portion prélevée, une quantité de prélèvement, et un type de cellule, et les informations de la culture des cellules incluent au moins des informations concernant une ligne de culture, une méthode de culture, une période de culture, un résultat d'évaluation de la qualité, un résultat d'évaluation de la validité d'un processus de culture, une image de cellule, et un résultat d'évaluation d'un environnement cellulaire, et
dans lequel les informations supplémentaires sont collectées et gérées sous la forme d'une seule information en
- stockant les informations sur un support de stockage tel qu'une étiquette d'identification par radiofréquence (RFID) prévue dans chaque récipient de culture, ou en
- stockant les informations sur un support de stockage prédéterminé conjointement avec des informations d'en-tête ou des informations similaires devant être lues sous la forme d'une seule information, ou en
- stockant les informations sur une zone de stockage unique d'un support de stockage prédéterminé.

15. Programme d'acquisition d'informations cellulaires, lequel lorsqu'il est exécuté sur un ordinateur (53) d'un système (1) selon l'une quelconque des revendications 1 à 13, fait en sorte que l'ordinateur (53) mette en œuvre le procédé selon la revendication 14.
